# EUROPÄISCHE PATENTSCHRIFT

(11) **EP 0 530 574 B1**
(45) Veröffentlichungstag und Bekanntmachung des Hinweises auf die Patenterteilung: **05.11.1997**
(21) Anmeldenummer: 92113972.1
(22) Anmeldetag: 17.08.1992
(51) Int. Cl.: A61C 1/00, A61B 17/36, G02B 6/42, H01S 3/109

(54) **Vorrichtung zur Lasermaterialbearbeitung biologischer Hartsubstanz, insbesondere Zahnhartsubstanz**
Apparatus for laser machining of high density bone in particular tooth enamel
Dispositif pour l'usinage par laser des matériaux biologiques à haute densité, en particulier l'email

(30) Priorität: 28.08.1991 DE 4128617
(43) Veröffentlichungstag der Anmeldung: 10.03.1993
(73) Patentinhaber: SIEMENS AKTIENGESELLSCHAFT, 80333 München (DE)
(72) Erfinder: Müller, Gerhard J., Prof. Dr., W-1000 Berlin (DE); Ertl, Thomas, W-1000 Berlin (DE)

(56) Entgegenhaltungen:
- EP-A- 0 073 617
- EP-A- 0 375 578
- EP-A- 0 392 951
- DE-A- 4 030 734
- FR-A- 2 598 313
- US-A- 3 620 597
- US-A- 4 736 743
- US-A- 4 808 789
- US-A- 4 909 609

## Beschreibung

Die Erfindung betrifft eine Vorrichtung zur Lasermaterialbearbeitung biologischer Hartsubstanz, insbesondere Zahnhartsubstanz.

### Stand der Technik

Vorrichtungen dieser Art sind beispielsweise aus DE-4 030 734 und DE-3 911 871 bekannt. In dem erstgenannten Dokument werden diverse Vorrichtungen beschrieben, mit denen mit Hilfe eines gepulsten Lasers, einem faseroptischen Übertragungssystem und einem faseroptischen Laserhandstück mit austauschbaren Therapieköpfen kariöse Zahndefekte behandelt und Wurzelkanalaufbereitungen durchgeführt werden können. Als Strahlungsquelle wird ein gepulster Alexandrit-Festkörperlaser verwendet, der im Wellenbereich zwischen 720 bis 860 nm arbeitet. Durch Zusatz eines geeigneten optischen Moduls kann eine Frequenzverdoppelung und damit ein Arbeitsbereich zwischen 360 und 430 nm erreicht werden. In einer der dargestellten Versionen tritt der Laserstrahl als Freistrahl aus dem abnehmbaren Kopfstück aus, d.h. der Fokus liegt außerhalb der Austrittsfläche. In einem anderen Ausführungsbeispiel erfolgt der Austritt am Ende eines in den Therapiekopf eingesetzten Lichtwellenleiters. Der Laserstrahl tritt hier divergent aus. Diese Version ist insbesondere zur Aufbereitung von Wurzelkanälen vorgesehen. Das Ein- und Auskoppeln aus Lichtleitern geschieht jeweils über Kugellinsen. Im Trennbereich enthalten Handstück und Therapiekopf stirnseitig jeweils entspiegelte Quarzglasfenster. Diese verhindern das Eindringen von Staub und Schmutz in die getrennten Einzelteile.

In dem anderen Dokument wird ein Verfahren zum Abtragen von Zahnmaterial mittels gepulstem Infrarot-Laser beschrieben, bei dem man das Zahnmaterial vor oder während der Bestrahlung mit einer dünnen Schicht einer die Laserstrahlung absorbierenden Flüssigkeit bedeckt. Dadurch kann die Gefahr einer Schädigung des umgebenden Gewebes gemindert werden, ohne gleichzeitig die Abtragungswirksamkeit zu beeinträchtigen. Die Flüssigkeit wird intermittierend vor jedem Laserpuls aufgetragen.

Es ist weiterhin allgemein bekannt, daß man mit gepulsten Lasersystemen bei Überschreiten einer materialspezifischen kritischen Energiedichte biologisches Material abtragen kann, ohne daß die Randzonen der Bearbeitungsstelle eine nennenswerte Temperaturerhöhung erfahren. Allerdings werden dabei extrem kurze Lichtimpulse im Nanosekundenbereich angewandt, und die abgetragenen Schichtdicken biologischen Materials bewegen sich dabei lediglich im Bereich von 10 - 50 µm. Um bei derartig geringen Schichttiefen je einzelnem Lichtimpuls zu nennenswerten Abtragraten zu gelangen, ist es notwendig, die Wiederholrate der Laserimpulse zu erhöhen. Da aber auch biologische Hartsubstanz nur ein endliches Wärmeleitvermögen hat, führt eine Erhöhung der Wiederholrate zu einem Wärmestau in der Umgebung der Abtragungsstelle und damit sehr schnell zu einer thermischen Schädigung der Randzonen der Bearbeitungsstelle. Zur Verringerung der thermischen Schädigung sind bei Lasersystemen, deren Strahlung von Wasser bzw. Luft nur gering absorbiert wird, Kühlungsvorrichtungen, die mit einem kontinuierlichen Wasserstrahl oder kontinuierlichen Luftstrom arbeiten, bekannt. Durch Untersuchungen von 'Srinivasan'ist es bekannt, daß es bei der Übertragung von Laserstrahlung mit hohen Lichtintensitäten über optische Fasern zu einem photohydraulischen Phänomen kommen kann, bei dem die laserinduziert ablatierten Kompartimente des biologischen Materials retrograd mit sehr hohen Geschwindigkeiten und kinetischen Energien auf die Endflächen der Übertragungsfaser einwirken, was sehr schnell zu einer Zerstörung der Lichtleitfasern und damit zum Abbruch des Bearbeitungsvorganges führt. Nach dem Stand der Technik wird die Übertragung derart hoher Lichtintensitäten meist über Spiegelgelenkarme vorgenommen. Eine solche Übertragung ist beispielsweise in der DE-A-39 11 853 beschrieben. Es wird ein optomechanisches Endstück für einen Spiegelgelenkarm eines Er:YAG-Lasers gezeigt, mit dem grundsätzlich die Übertragbarkeit gepulster Laserstrahlung realisiert werden kann. Derartige Spiegelgelenksysteme haben allerdings den Nachteil, daß jeder Fehler bei der Montage bzw. in der Stabilität der Ausgangsrichtung der Laserstrahlung an jedem Umlenkspiegel verdoppelt wird und insoweit Abweichungen der Koaxialität durch Fehler der Einkoppeloptik bzw. dynamische Wanderung der Ausgangsrichtung der Laserstrahlung oder Änderung seines Divergenzwinkels mit dem Faktor 2 hoch Anzahl der Spiegelumlenkungen zu multiplizieren ist. Dadurch sind derartige Spiegelgelenksysteme äußerst justierempfindlich und bedingen einen extrem hohen mechanischen Herstellungsaufwand. Hinzu kommt, daß solche Spiegelgelenksysteme nur bedingt frei manövrierbar sind, da zu Artikulationen einer jeden Umlenkung Kräfte aufgebracht werden müssen, und zwei beliebige Raumpunkte nicht immer auf dem kürzesten Weg miteinander verbunden werden können.

Es ist nach dem Stand der Technik ferner auch bekannt, optische Strahlung über Lichtwellenleiter zu übertragen. Allerdings zeigt sich, daß mit den derzeit verfügbaren Materialien für optische Fasern eine Übertragbarkeit hochintensiver kurzer Laserimpulse nur bedingt möglich ist.

Aus der US-A-4,909,609 ist es bekannt, zum Schutz der Augen vor schädlichen Laserstrahlen in die Strahltaille eines durch optische Elemente auf einen Fokus konzentrierten Laserstrahles ein Kristall mit nichtlinearen Eigenschaften einzusetzen, mit dem sich die Grundfrequenz von Strahlung hoher Leistungsdichte verdoppeln und damit die Intensität auf eine niedrigere Größe reduzieren läßt.

### Aufgabenstellung

Es soll eine Vorrichtung angegeben werden, mit der es möglich ist, biologische Hartsubstanz, wie z. B. Knochen oder Zähne, in einer Abtragrate, die mit dem konventionellen, in der Dentaltechnik üblichen Bohren oder Fräsen, vergleichbar ist, zu bearbeiten, ohne daß dabei in der Randzone der Bearbeitung thermisch geschädigte Zonen entstehen, die größer sind als die optische Eindringtiefe der verwendeten Laserstrahlung. Die Übertragung der Laserstrahlen vom Lasergenerator auf das Bearbeitungshandstück und von dort auf das Ablationsobjekt soll dabei möglichst ohne Effektivitätsverlust ablaufen.

### Erfindung

Durch die erfindungsgemäß vorgeschlagenen Maßnahmen werden die während der Dauer eines Impulses auftretenden Spitzen (Spikes), die im Verhältnis zur Laserpulslänge kurze Überhöhungen der abgegebenen Laserenergie sind und oberhalb einer bestimmten Intensitätsschwelle liegen, geglättet. Das Glätten kann vorteilhafterweise durch Frequenzverdoppelung erzielt werden, wobei nur die Frequenzen der Intensitätsspitzen verdoppelt werden. Die Grund- oder Arbeitswellenlänge, mit der der Laser betrieben wird, bleibt davon unberührt.

Es hat sich überraschenderweise gezeigt, daß die Volumenzerstörschwelle optischer Fasern deutlich über der Oberflächenzerstörschwelle bei Beaufschlagung mit hochintensivem Laserlicht liegt, wobei die Oberflächenzerstörschwelle etwa proportional zur Wurzel aus der Pulslänge ansteigt. Das bedeutet, daß sich im Grenzfall eines kontinuierlich strahlenden Lasers die Oberflächen- und Volumenzerstörschwellen bis auf Effekte höherer Ordnung einander annähern. Um der Forderung möglichst geringer Schädigungsbreiten in der Randzone des Bearbeitungsgebietes zu genügen, kommen daher nur Laser mit Emissionswellenlängen entweder < 400 nm oder > 1,1 µm in Betracht. Unter Beachtung der weiteren Randbedingungen gemäß Aufgabenstellung, daß die Abtragungsraten vergleichbar konventionellen Systemen sein müssen, kommen lediglich kurzgepulste Laser mit Pulslängen zwischen ca. 0,5 und 500 µs in Betracht. Unter Beachtung des Absorptionsverhaltens der Zahnhartsubstanzen bzw. des Knochens kommen als Arbeitswellenlängen 200 bis 400 nm, 1,3 bis 3 µm und 9,0 bis 11 µm in Frage. In einem bevorzugten Ausführungsbeispiel wird ein Festkörperlaser mit Emissionswellenlänge 2,78 µm, vornehmlich ein Cr-Er-dotierter YSGG-(Yttrium Scandium Gadolinium Granat) Kristall verwendet. Alternativ kann vorteilhafterweise ein Er:YAG-Laser, der Strahlung im Wellenlängenbereich bei 2,96 µm emittiert, eingesetzt werden.

Mit den folgend näher beschriebenen Maßnahmen macht man sich das auch für den Fachmann völlig überraschende Ergebnis zunutze, daß durch den Ablationsprozeß selbst während der Einwirkdauer eines gepulsten Lasers das Absorptionsmaximum der Zielsubstanz sich dynamisch zu kürzeren Wellenlängen verschiebt. In dem, dem bevorzugten Ausführungsbeispiel zugrundeliegenden Anwendungsfeld der Bearbeitung von Zahnhartsubstanz verschiebt sich das Absorptionsmaximum von wasserhaltiger Zahnhartsubstanz (Hydroxylapatit) von etwa 3 µm auf etwa 2,8 µm, d.h. bei Benutzung der bevorzugten Wellenlänge optimiert sich der Prozeß während jedes einzelnen Impulses selbst.

Mehrere Ausführungsformen der Erfindung werden nachfolgend anhand der Zeichnung näher beschrieben.

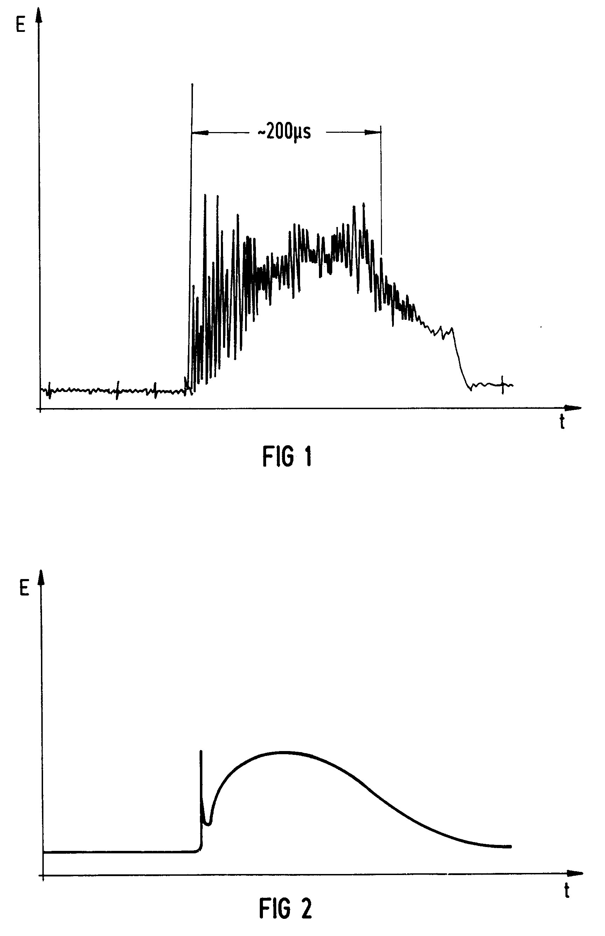

Figur 1 zeigt das zeitliche Emissionsverhalten eines gepulsten Festkörperlasers. Durch die gegenüber der mittleren Energie stark überhöhten Spitzen treten sehr hohe Leistungsdichten bei der Einkopplung dieser Strahlung an der Oberfläche von Fasern auf, und selbst, wenn die entstehenden Leistungsdichten noch zu gering sind, um zu einer Volumenzerstörung der Fasern zu führen, wird jedoch in aller Regel die Oberfläche bereits bei der Einkopplung der Strahlung zerstört und führt damit zum Versagen des Systems.

Figur 2 zeigt ein für die Übertragung von hohen Pulsenergien ideales Emissionsverhalten, wie es durch die erfindungsgemäßen Maßnahmen erzielt wird.

Die Lösung des technischen Problems der leichten Übertragbarkeit entsprechend gepulster Lasersystem über optische Fasern wird erfindungsgemäß dadurch gelöst, daß in die Strahltaille des Lasers, entweder innerhalb oder außerhalb des Resonators, ein Kristall mit nichtlinearen optischen Eigenschaften, d.h. Erzeugung des Vielfachen der Grundfrequenz, gestellt wird, der oberhalb bestimmter Grenzwerte der Leistungsdichte mit hoher Effizienz die Harmonischen der Grundwellenlänge generiert. Als Kristall kann Lithium-Jodat,, Lithium-Niobat oder Barium-Betaborat zur Anwendung kommen. Durch weitere optische Maßnahmen oder durch zusätzliche optische Elemente wird erreicht, daß die Strahltaille eine solche Leistungsdichte für die chaotischen Emissionsimpulse der Laserstrahlung aufweist, daß oberhalb einer bestimmten Leistungsdichte, die dem Mittelwert eines Impulses entspricht, die Intensitätsspitzen frequenzverdoppelt werden, wodurch diese nicht weiter am Verstärkerprozeß teilnehmen.

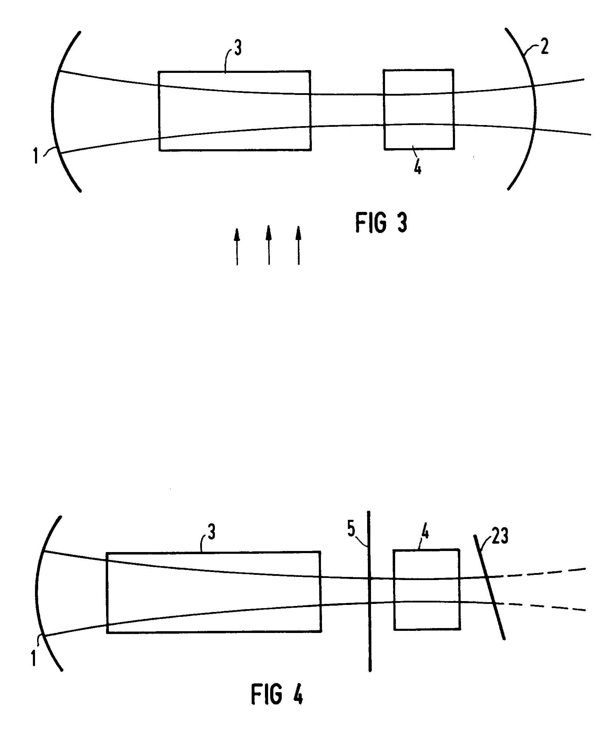

Bei der in Figur 3 dargestellten Anordnung wird der Resonator aus zwei konfokalen (konvexgekrümmten) Spiegeln 1, 2 gebildet, zwischen denen ein mit 3 bezeichnetes laseraktives Medium (Gas, Flüssigkeit, Festkörper oder Halbleiter) angeordnet ist. Innerhalb des Resonators ist ein mit 4 bezeichneter Kristall angeordnet, der die vorgenannten Eigenschaften aufweist. Bei Benutzung dieser Erkenntnisse innerhalb des Resonators werden wegen der frequenzselektiven Verstärkungseigenschaften des Laserkristalls die Harmonischen nicht weiter verstärkt, so daß lediglich die Impulsspitzen durch diesen Prozeß gekappt werden, jedoch die Grundwellenlänge weiterhin verstärkt wird. Eine Anordnung dieser Art innerhalb des Resonators führt zu geringstmöglichen Verlusten.

Im Rahmen der Erfindung ist jedoch auch eine Anordnung des Kristalls 4 außerhalb des Resonators möglich, wie dies in Figur 4 am Beispiel eines Resonators mit teilverspiegeltem Planspiegel 5 gezeigt ist. Mit Vorteil können bei dieser Ausführungsform in einfacher Weise die Bedingungen zur Erzeugung der Harmonischen leichter eingestellt und variiert werden. Mit 23 ist ein dichroitischer Strahlenteiler bezeichnet, mit dem sich die Intensitätsspitzen der frequenzverdoppelten Laserpulse ausspiegeln lassen. Mit einem derart geglätteten Intensitätszeitprofil eines gepulsten Er:YSSG-Lasers bzw. eines ER:YAG-Lasers können dann über nach dem Stand der Technik verfügbare optische Fasern Pulsenergien von mehr als 500 mJ bei Impulsbreiten von ca. 180 µs übertragen werden. Erfindungsgemäß kann jedoch auch jeder andere nichtlineare optische Mechanismus zur Beschneidung der Impulsspitzen verwendet werden, um eine sichere Einkopplung hochintensiver Lichtimpulse zu gewährleisten. Als Beispiel sei hier ein Zweiphotonenabsorber genannt.

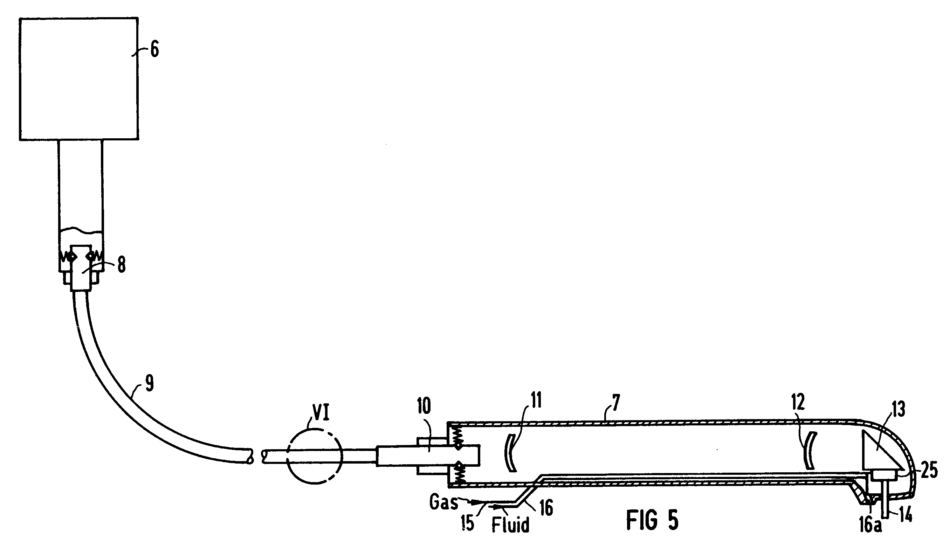

Anhand der Figur 5 wird die Führung des Laserlichts von einem allgemein mit 6 bezeichneten Lasergenerator zu einem zahnärztlichen Handstück 7 aufgezeigt. Der Lichtaustrittsstrahl am Resonator des Lasergenerators 6 wird mit Hilfe einer nicht näher dargestellten Einkoppeloptik auf das Zentrum einer das Licht übertragenden ersten Drehkupplung 8 gerichtet und wird dort mittels eines flexiblen Lichtwellenleiterschlauches 9 bis zu einer zweiten Drehkupplung 10 geführt, an die das Handstück 7 leicht lösbar angekuppelt ist. Von der zweiten Drehkupplung 10 aus wird das Laserlicht als Freistrahl über optische Spiegel und Prismen 11, 12 und 13 schließlich zu einem Applikatorendstück 14, dem eigentlichen Arbeitswerkzeug, geleitet. Mit 15, 16 sind zwei später noch näher erläuterte Medienleitungen bezeichnet, über die einerseits Gas, vorzugsweise Luft, und andererseits ein Fluid, vorzugsweise Wasser, an das Kopfteil des Handstückes herangeführt wird. Die Gasleitung 15 ist einerseits mit einem später noch näher erläuterten Gasbehälter 25 und andererseits über eine Abzweigleitung mit der Fluidleitung 16 verbunden, deren mit 16a bezeichnete Austrittsdüse auf die Arbeitsstelle so ausgerichtet ist, daß Aerosol gezielt in die Wirkzone der Laserstrahlung, also nicht in deren Umgebung, eingesprüht werden kann.

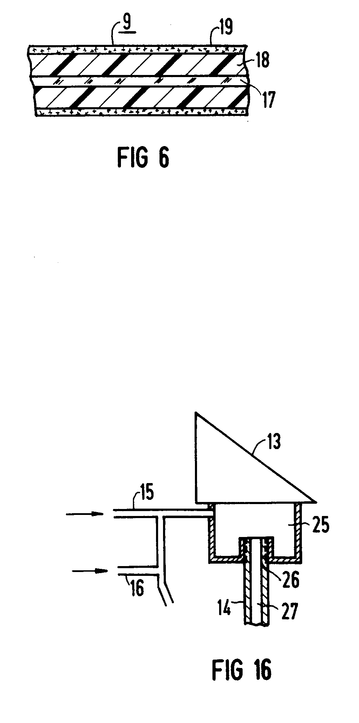

Wie anhand der Figur 6, die einen Ausschnitt vom Lichtwellenleiterschlauch 9 im Längsschnitt zeigt, ersichtlich, ist der Lichtwellenleiter 17 zentrisch angeordnet.

In dem bevorzugten Ausführungsbeispiel wird als Material für den Lichtwellenleiter Zirkonium-Fluoridglas verwendet. Der Lichtwellenleiter kann sowohl aus einer Faser wie auch aus einer Multifaseranordnung (Faserbündel) bestehen. Dabei handelt es sich zwar um einen biegeelastischen Stoff, der andererseits aber sehr torsionsempfindlich ist. Daher ist bei Konstruktion einer entsprechenden Vorrichtung erfindungsgemäß dafür Sorge getragen, daß Torsionskräfte gar nicht bzw. nur in äußerst geringem Umfange auf den optischen Lichtwellenleiter übertragen werden. Der Lichtwellenleiter 17 ist deshalb in einem elastischen Kunststoffmantel 18 eingebettet, der bei seinen viskoelastischen Eigenschaften auftretende Scher- und Tosionskräfte auf größere Oberflächenelemente verteilt. In einem bevorzugten Ausführungsbeispiel handelt es sich dabei um Silikonkautschuk. Der derart mit Silikonkautschuk ummantelte optische Wellenleiter ist in einem biegeelastischen äußeren Hüllschlauch 19, der durch das Einbringen von Gewebelagen torsionssteif ausgeführt ist, angeordnet. An beiden Enden befinden sich die bereits erwähnten Drehkupplungen 8, 10, wobei diese so ausgelegt sind, daß sie sich im jeweiligen Gegenstück, also zum einen auf der Laserseite, zum anderen auf der Applikatorseite, um die Längsachse frei drehen können.

Durch die erfindungsgemäß vorgeschlagenen Maßnahmen zur Reduktion der Oberflächenzerstörwahrscheinlichkeit eines optischen Wellenleiters mit Übertragungseigenschaften für Er:YSSG- bzw. ER;YAG-Laserstrahlung und Maßnahmen zur Vermeidung auf den Lichtwellenleiter werden die eingangs angestrebten Ziele erreicht.

Die Forderung nach einem sterilisierbaren, optisch mechanischen Endstück wurde erfindungsgemäß folgendermaßen gelöst:

Die aus dem flexiblen Lichtwellenleiter 17 austretende gepulste Strahlung des bevorzugten Er:YSGG- oder aber auch des Er:YAG-Lasers wird über die Rotationsschnellkupplung 10 in das röhrenförmige Handstück 7 geführt. Dadurch kann das Handstück zur Sterilisation vom optischen Wellenleiter getrennt werden. Die Sterilisierbarkeit wird erreicht durch Verwendung von nur wenigen optischen Bauelementen, die nicht gekittet sind.

Gemäß den Figuren 5 sowie 7 bis 12 wird innerhalb des Handstückes die Strahlung über geeignete optische Elemente 11 bis 13 sowie 20 bis 21 in das Applikatorendstück 14 geführt. Alle in den Abbildungen gezeigten Handstücke zeigen den Vorteil eines auswechselbaren Applikatorendstücks, das in einfacher Weise steril geliefert bzw. sterilisiert werden kann.

Figur 5 zeigt die Möglichkeit, das Licht über zwei konjugierte Abbildungslinsen 11, 12, die vorzugsweise als Meniskuslinsen aus Saphir ausgeführt sind, zu leiten, wobei eine Abbildung der Austrittsapertur des flexiblen Lichtwellenleiters 17 über das Umlenkprisma 13 in die Eintrittsapertur des als Hohlleiter ausgebildeten Applikatorendstücks 14 erzielt wird. Das Umlenkprisma ist derart in den Strahlengang der Relaisoptik 11, 12 gestellt, daß an der Hypothenusenfläche des Prismas Totalreflexion für den Winkelbereich der Laserstrahlung auftritt. So wird eine Strahlumlenkung um etwa 90° erreicht. Wie in Figur 16 vergrößert dargestellt, sitzt das Prisma 13 erfindungsgemäß auf einem Gasbehälter 25 auf, in den über die Leitung 15 Gas, vorzugsweise Luft, eingeleitet wird. Der Gasbehälter 25 ist mit einem Gewindeanschluß 26 versehen, wodurch es möglich ist, Applikatorendstücke 14 verschiedener Länge und Öffnungsverhältnisse einsetzen bzw. auswechseln zu können. Das Applikatorendstück 14 ist hier als Hohlleiter ausgebildet; über den mit dem Gasbehälter 25 verbundenen Kanal 27 kann ein Teil des zur Aerosolbildung verwendeten Druckgases ausgetrieben werden. Durch den Gasbehälter wird eine gleichmäßige Druckverteilung des Gasflusses durch den Hohlleiter erreicht, was sodann bei einem Verhältnis Innendurchmesser zu Länge der Strömungskapillare von mindestens 1:10 zu einem laminaren Fluß führt. Dadurch wird erfindungsgemäß erreicht, daß die beim Interaktionsprozeß der kurz gepulsten Laserstrahlung mit Materie rückwärts herausgeschleuderten Partikel durch den Gegenstrom des Gases nicht die optischen Flächen der Umlenkelemente 13, 20 und 21 erreichen können, sondern durch den Gasstrom seitlich ausgetrieben werden. Damit sind die empfindlichen Flächen des Applikatorendstückes 14 sowie der Umlenkelemente vor Beschädigung durch Ablationsprodukte gasdynamisch geschützt. Gleichzeitig wird bei Verwendung eines höherbrechenden Gases als Luft über eine kurze Distanz am Ende des Hohlleiters auch noch eine Wellenleiteigenschaft im Gasstrahl für die verwendete Wirkstrahlung erreicht, wobei ein Arbeiten im Nonkontakt möglich ist. Gleichzeitig wird dadurch der Zugang der Aerosol-Spülung zum Arbeitsfeld erreicht.

In einem bevorzugten Ausführungsbeispiel ist als Material für den auswechselbaren Applikator 14 eine innen vergoldete Kapillare verwendet, die, wie ein Bohrer im Handstück einer Zahnarztturbine, ausgewechselt werden kann.

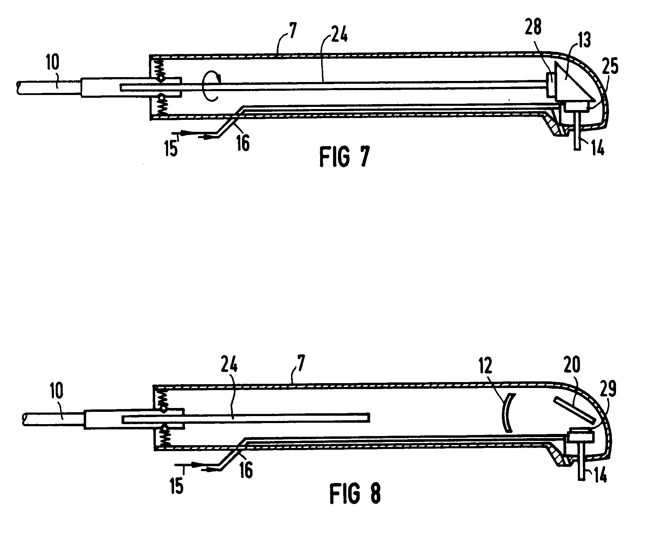

Die Ausführungen nach den Figuren 7 und 8 sehen alternativ zur Benutzung einer Relaisoptik die Weiterführung des optischen Wellenleiters 17 in einer steifen, mit der Drehkupplung verbundenen Metallhülse 24 bis nahe an das proximale Ende des Umlenkelements vor, das entsprechend Figur 7 aus einem optischen Prisma 13 oder entsprechend Figur 8 aus einem Umlenkspiegel 20 bestehen kann. Zur Vermeidung von Reflexionsverlusten ist vorgesehen, zwischen der Endfläche des Lichtwellenleiters 17 und der Umlenkeinheit Immersionsflüssigkeit einzufügen, die im Ausführungsbeispiel in einer Kapsel 28, in der das Ende der Metallhülse 24 geführt ist, enthalten ist. Für den Fall, daß die numerische Apertur der Faser hinreichend klein ist, d.h. kleiner 0,2, kann die austretende Strahlung unmittelbar über das Umlenkelement in den auswechselbaren Hohlleiter geführt werden.

Figur 8 zeigt ein Ausführungsbeispiel, bei dem als Umlenkelement ein Planspiegel 20 verwendet ist.

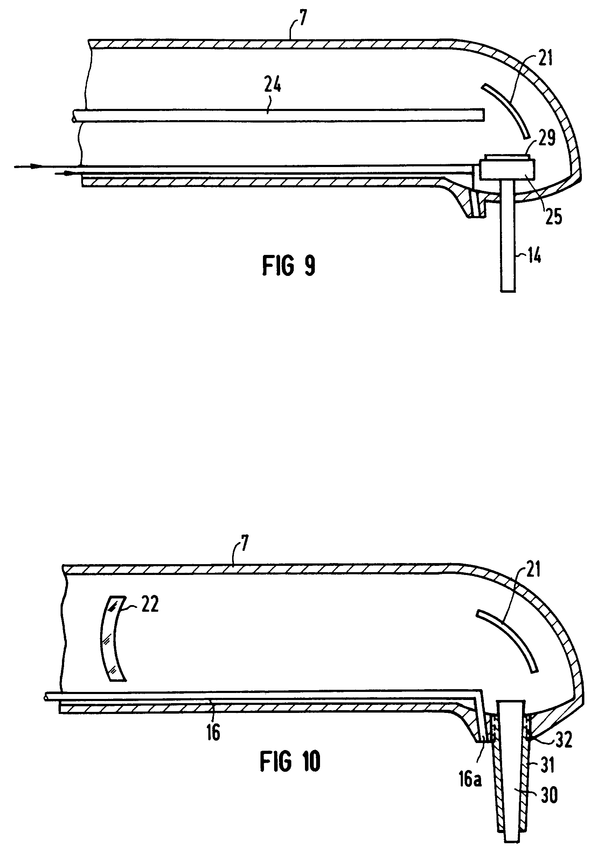

Für den Fall, daß die numerische Apertur des Lichtwellenleiters zu groß ist, so daß bei den vorhandenen geometrischen Abständen die Strahlung nicht mehr vollständig in den auswechselbaren Hohlleiter umgelenkt werden kann, ist für das Umlenkelement in einem bevorzugten Ausführungsbeispiel gemäß Figur 9 ein torischer Spiegel 21 voresehen, der eine der Eintrittsapertur des Hohlleiters entsprechende Konvergenz der Strahlung herbeiführt. Gleichzeitig ist in diesem Falle der Gasbehälter 25 durch ein optisches Fenster 29 abgeschlossen und ermöglicht wiederum auf der gegenüberliegenden Seite die leichte Auswechselbarkeit des optischen Hohlleiters 14. Als bevorzugte Ausführung des optischen Fensters ist eine dünne Glimmerscheibe vorgesehen.

In einem weiteren Ausführungsbeispiel gemäß Figur 10 besteht die Relaisoptik aus einer Linse 22, vorzugsweise einer Meniskuslinse, in Kombination mit einem torischen Spiegel 21, wobei in diesem Falle die Strahlung in eine konische Saphirspitze 30 eingekoppelt wird, die sich aus Schutz vor Verschmutzung in einem Metallkonus 31 befindet. Der Metallkonus 31 ist mittels Schraubgewinde im Kopfgehäuse des Handstückes 7 leicht auswechselbar gehaltert. In diesem Falle wird ausgenutzt, daß Saphir aufgrund seiner Härte, aber guten optischen Transparenz, für die Nutzwellenlängen als Lichtwellenleiter genutzt werden kann, der am distalen Ende durch die rückgeschleuderten Partikel nur wenig beschädigt wird, daß aber für den Fall einer zunehmenden Beschädigung durch die leichte Auswechselbarkeit der Spitze ein schneller Ersatz möglich ist, ähnlich wie es heute der Zahnarzt beim Auswechseln des Bohrers gewöhnt ist.

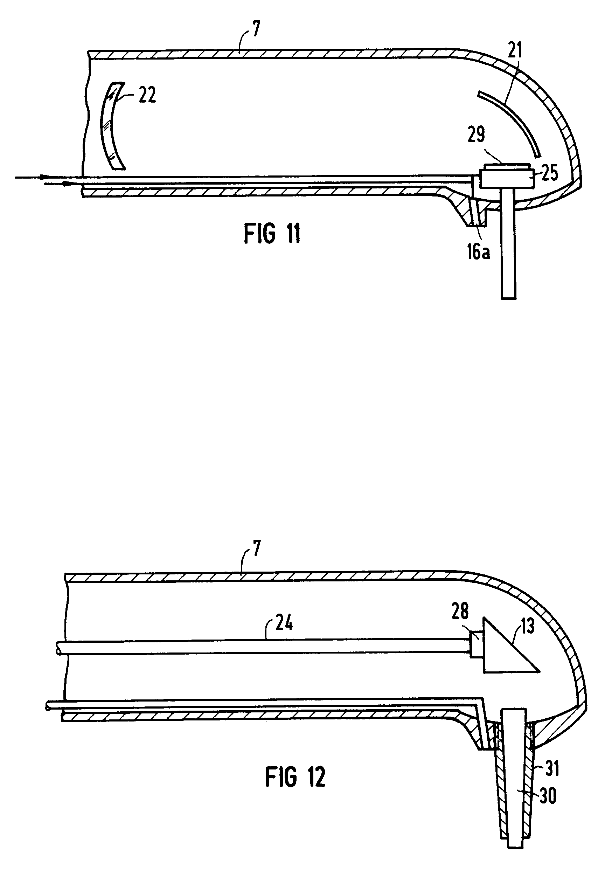

Bei Nutzung einer Relaisoptik (Fig. 10), bestehend aus Meniskuslinse 22 und torischem Spiegel 21 kann selbstverständlich auch ein auswechselbarer Hohlleiter mit gasdynamischem Schutz verwendet werden, wie dies in Figur 11 gezeigt ist.

Ebenso kann selbstverständlich anstelle der Relaisoptik im Falle des Saphirapplikators auch der in der Metallhülse 24 geschützt angeordnete Lichtwellenleiter bis zum Umlenkelement geführt sein, das entweder als Prisma oder torischer Spiegel ausgeführt ist, wie dies in Figur 12 gezeigt ist.

Das Verfahren zur Abtragung von biologischer Hartsubstanz, wie Knochen oder Zahnmaterial mit Laserstrahlung, sieht vor, daß die Wirkzone der Laserstrahlung intermittierend mit Aerosol-Tropfen beaufschlagt wird, die durch den Abtragungsprozeß selbst ebenfalls mit entfernt werden, aber aufgrund der Verdampfungswärme der Aerosol-Tropfen der Umgebung so viel Wärme entziehen, daß bei Wiederholraten bis zu 15 Hz gearbeitet werden kann, ohne daß die thermisch geschädigte Übergangszone größer ist als z.B. bei Anwendung eines Turbinenbohrers. Dabei konnte überraschenderweise gefunden werden, daß bei Einhaltung einer bestimmten Luft-Fluid-Relation und einer an die Pulsenergie und Wiederholrate angepaßte Fluidmenge kein Effektivitätsverlust auftrat. Zudem wird eine wesentliche qualitative Verbesserung des Ablationsprozesses erreicht.

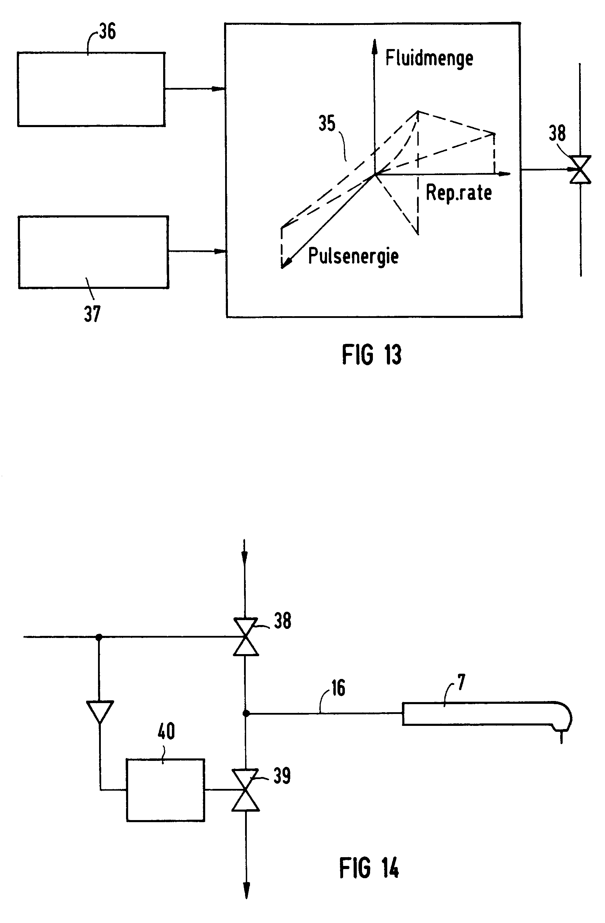

Durch diese Maßnahmen konnte erstmals eine Ablation von Hartgewebe am Punkt höchster Effektivität bei Einhaltung der engen Temperaturgrenzen (< 5K Temperaturerhöhung) erreicht werden. Anhand der Figuren 13 bis 15 wird dies näher erläutert.

Figur 13 zeigt die erfindungsgemäße Anpassung der Luft-Fluid-Relation des Aerosols sowie des Gesamtfluidanteils an die Laserpulsenergie und die Wiederholrate der Laserpulse. Dazu wird in einem PROM / EPROM eines Mikrocontrollers 34 ein Kenndatenfeld 35 als Matrix abgelegt. Der Mikrocontroller 34, dem Informationen über Pulsenergie (36), Fluidmenge und Repetitionsrate (37) zugeführt werden, steuert ein Ventil 38 zur Fluidmengenregulation.

Um den Applikator 14 möglichst klein und handlich gestalten zu können, wurde in dem bevorzugten Ausführungsbeispiel das Ventil 38 vor der Zuführung zum Applikator 14 eingesetzt. Damit es nicht aufgrund der Dehnbarkeit des Zuführungsschlauches 15 zum Nachlaufen des Fluids kommt, wird der Wasserzuführungsschlauch phasenversetzt mit Unterdruck beaufschlagt. In dem bevorzugten Ausführungsbeispiel wurde gemäß Figur 14 ein zweites Ventil 39 invertiert zu Ventil 38 angesteuert. Das entsprechende Steuerteil ist mit 40 bezeichnet. Wird ein nicht dehnbarer Zuführungsschlauch verwendet, genügt die Druckentlastung des Schlauchsystems über das Ventil 39. Die Beaufschlagung mit Unterdurck kann dann entfallen.

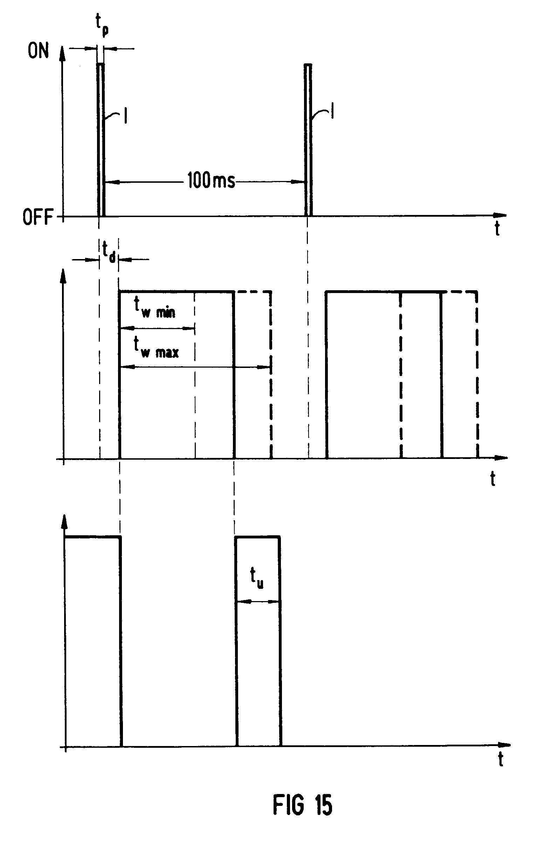

Figur 15 zeigt das Timing Diagramm zur Steuerung des Aerosols. In dem Diagramm sind untereinander die Betriebszustände (ON, OFF) für die Laserimpulse, das Ventil 38 sowie das Unterdruckventil 39 dargestellt. Aus der Darstellung ist ersichtlich, daß das Fluidventil 38 phasenverschoben um t_{d} zu den Laserpulsen J angesteuert wird, um die Energieverluste innerhalb des Aerosols während des Laserpulses möglichst klein zu halten. Die Fluidmenge wird über das Puls-Pause-Verhältnis gesteuert (t_{Wmin}, t_{Wmax}). Das Unterdruckventil 39 wird über einen Monoflop des Steuerteils 40 für eine definierte Zeit tᵤ eingeschaltet, die ausreicht, um die Fluidsäule im Zuführungsschlauch abzubremsen.

In der bevorzugten Ausführungsform bei Verwendung eines pulsgeformten Er:Cr:YSGG-Lasers ist das Fluid reines Wasser. Erfindungsgemäß kann jedoch auch jedes andere für den jeweiligen Anwendungsfall geeignete Fluid mit hinreichend hoher optischer Absorption bei der Arbeitswellenlänge des Lasers benutzt werden. Gleichzeitig sorgt die Verwendung eines optischen Hohlleiters mit Gasspülung für einen Schutz der empfindlichen optischen Komponenten. Außerdem ergeben sich die Vorteile einer einfachen Handhabbarkeit und leichter Sterilisierbarkeit.

## Patentansprüche

1. Vorrichtung zur Ablation biologischer Hartsubstanz, insbesondere Zahnhartsubstanz, **dadurch gekennzeichnet,** daß ein kurzgepulster Festkörperlaser verwendet ist, der Strahlung im Wellenlängenbereich zwischen 2,78 und 2,94 µm emittiert und daß zur Glättung des Intensitätszeitverhaltens des Festkörperlasers in die Strahltaille des Lasers, innerhalb oder außerhalb des Laserresonators (1, 2; 1,5) ein Kristall (4) mit nichtlinearen optischen Eigenschaften zur Erzeugung der ersten Harmonischen eingebracht ist, wobei die Strahltaille eine solche Leistungsdichte für die chaotischen Emissionsimpulse der Laserstrahlung aufweist, daß oberhalb bestimmter Grenzwerte der Leistungsdichte, die dem Mittelwert eines Impulses entspricht, die Intensitätsspitzen eines Laserpulses durch Erzeugung eines Vielfachen der Grundfrequenz, vorzugsweise durch eine Frequenzverdoppelung, geglättet werden, und daß bei Anordnung des Kristalls außerhalb des Resonaters Mittel (23) vorgesehen sind, mit denen die in ihrer Frequenz vervielfachten Intensitätsspitzen aus dem Strahlverlauf der in den Lichtwellenleiter einzukoppelnden Grundwellenlänge entfernt werden.

2. Vorrichtung nach Anspruch 1, **dadurch gekennzeichnet,** daß der in den Einkoppelstrahlengang des Festkörperlasers angeordnete Kristall (4) außerhalb des Laserresonators (1,5) und in Bezug zu einem Lichtwellenleiter (17) derart angeordnet ist, daß die Intensitätsspitzen eines Laserpulses frequenzverdoppelt und über einen dichroitischen Strahlteiler (23) aus dem Strahlengang entfernt werden, so daß in den Lichtwellenleiter (17) lediglich die Grundwellenlänge eingekoppelt wird, die dann zu keinem Zeitpunkt mehr bestimmte kritische Leistungsdichten überschreitet.

3. Vorrichtung nach Anspruch 1, **dadurch gekennzeichnet,** daß als Laser ein bei 2,78 µm Wellenlänge arbeitender Cr-Er-dotierter Yttrium-Scandium-Gadolinium-Granat verwendet wird.

4. Vorrichtung nach Anspruch 1, **dadurch gekennzeichnet,** daß als Laser ein bei 2,94 µm arbeitender Er:YAG-Laser verwendet wird.

5. Vorrichtung nach Anspruch 2, **dadurch gekennzeichnet,** daß als Kristall (4) Lithium-Jodat oder Silber-Gallium-Sulfid verwendet wird.

6. Vorrichtung nach Anspruch 1, **dadurch gekennzeichnet,** daß die gepulste Wirkstrahlung durch einen optischen Lichtwellenleiter (17) in Form einer Faser oder einer Multifaseranordnung aus Zirkonium-Fluorid übertragen wird.

7. Vorrichtung nach Anspruch 6, **dadurch gekennzeichnet,** daß der Lichtwellenleiter (17) mit einem viskoelastischen Kunststoff (18) umgeben und in einen torsionsteifen Hüllschlauch (19), eingebettet ist.

8. Vorrichtung nach Anspruch 6, **dadurch dadurch gekennzeichnet,** daß der Lichtwellenleiter (17) laser- und applikatorseitig mit einer Rotationsschnellkupplung (8, 10) versehen ist, die das Einbringen von Torsionskräften auf den flexiblen Teil des Lichtwellenleiters vermeidet.

9. Vorrichtung nach Anspruch 6, bei dem der Lichtwellenleiter (17) mit einem Handstück verbindbar ist, welches lichtaustrittsseitig ein Applikatorendstück (14) aufweist, **dadurch gekennzeichnet,** daß in den Strahlengang des Laserlichts Mittel (25, 29, 30) vorgesehen sind, die verhindern, daß die durch die Strahlung erzeugten Ablationsprodukte nicht ihrerseits die optischen Flächen des Applikatorendstückes (14) zerstören können.

10. Vorrichtung nach Anspruch 9, **dadurch gekennzeichnet,** daß das Applikatorendstück (14) als optischer Hohlleiter ausgebildet ist, der mit einem an eine Gaszufuhrleitung (15) angeschlossenen Gasbehälter (25) verbunden ist.

11. Vorrichtung nach Anspruch 10, **dadurch gekennzeichnet,** daß der Gasbehälter (25) zumindest laserseitig durch ein optisches Fenster (29), vorzugsweise aus Glimmer, verschlossen ist.

12. Vorrichtung nach Anspruch 11, **dadurch gekennzeichnet,** daß das Applikatorendstück (14) eine vorzugsweise konisch geformte Saphirspitze (30) ist.

13. Vorrichtung nach Anspruch 1, **dadurch gekennzeichnet,** daß Mittel (15, 16, 38, 39) vorhanden sind, mit deren Hilfe Aerosol-Tröpfchen in die Wirkzone der Laserstrahlung eingeblasen werden können und daß weitere Mittel (34 bis 40) vorhanden sind, mit deren Hilfe die Luft-Fluid-Relation und die Fluidmenge in Abhängigkeit von der Laserpulsenergie und Pulswiederholrate gesteuert werden können.

## Claims

1. Device for the ablation of biological hard material, in particular the hard material of the teeth, characterized in that a short-pulsed solid-state laser is used which emits radiation in the wavelength range between 2.78 and 2.94 µm and in that to smooth the intensity time response of the solid-state laser into the beam waist of the laser, inside or outside the laser resonator (1, 2; 1,5) a crystal (4) with non-linear optical properties is introduced in order to generate the first harmonic, whereby the beam waist has such a power density for the chaotic emission pulses of the laser radiation that above certain limiting values of the power density which corresponds to the mean value of a pulse, the intensity peaks of a laser pulse are smoothed by producing a multiple of the fundamental frequency, preferably by doubling the frequency, and in that with an arrangement of the crystal outside the resonator there are provided means (23) with which the intensity peaks, the frequency of which is multiplied, are removed from the beam course of the fundamental wavelength to be coupled into the optical waveguide.

2. Device according to claim 1, characterized in that the crystal (4) arranged in the coupling beam path of the solid-state laser is arranged outside the laser resonator (1,5) and with respect to an optical waveguide (17) in such a way that the frequency of the intensity peaks of a laser pulse is doubled and the peaks are removed from the beam path by way of a dichroitic beam splitter (23) so that only the fundamental wavelength is coupled into the optical waveguide (17), which wavelength then no longer exceeds certain critical power densities.

3. Device according to claim 1, characterized in that a Cr-Er-doped yttrium-scandium-gadolinium garnet operating at 2.78 µm wavelength is used as laser.

4. Device according to claim 1, characterized in that a Er:YAG laser operating at 2.94 µm is used as laser.

5. Device according to claim 2, characterized in that lithium-iodate or silver-gallium sulphide is used as crystal (4).

6. Device according to claim 1, characterized in that the pulsed active radiation is transmitted by an optical waveguide (17) in the form of a fibre or a multi-fibre arrangement of zirconium fluoride.

7. Device according to claim 6, characterized in that the optical waveguide (17) is surrounded by elastic-viscous plastics (18) and is embedded in a torsion-proof encasing tube (19).

8. Device according to claim 6, characterized in that the optical waveguide (17) is provided on the laser and applicator side with a rapid rotation coupling (8, 10) which avoids the introduction of torsional forces on to the flexible part of the optical waveguide.

9. Device according to claim 6, where the optical waveguide (17) can be connected to a handpiece which has an applicator end piece (14) on the side of the light outlet, characterized in that in the beam path of the laser light there are provided means (25, 29, 30) which prevent the ablation products, generated by the radiation, from not [sic] being able to destroy on their part the optical surfaces of the applicator end piece (14).

10. Device according to claim 9, characterized in that the applicator end piece (14) is constructed as an optical hollow conductor which is connected to a gas container (25) attached to a gas supply line (15).

11. Device according to claim 10, characterized in that the gas container (25) is sealed at least on the side of the laser by an optical window (29), preferably comprising mica.

12. Device according to claim 11, characterized in that the applicator end piece (14) is a preferably conically formed sapphire tip (30).

13. Device according to claim 1, characterized in that means (15, 16, 38, 39) are present, with the aid of which aerosol droplets can be blown into the active zone of the laser radiation and in that additional means (34 to 40) are present, with the aid of which the relation between air and fluid and the fluid quantity can be controlled as a function of the laser pulse energy and the pulse repetition rate.

## Revendications

1. Dispositif d'ablation d'une substance biologique dure, notamment d'une substance dentaire dure, caractérisé en ce qu'il est utilisé un laser solide à impulsions courtes, qui émet un rayonnement dans la plage de longueurs d'ondes comprise entre 2,78 et 2,94 µm, un cristal (4) à propriétés optiques non linéaires est monté pour la production du premier harmonique pour le lissage du comportement temporel de l'intensité du laser solide dans la section transversale du rayonnement du laser, à l'intérieur ou à l'extérieur du résonateur laser (1, 2 ; 1,5), la section transversale de rayonnement ayant une densité de puissance telle, pour les impulsions chaotiques d'émission du rayonnement laser, que les pics d'intensité d'une impulsion laser soient lissés par production d'un multiple de la fréquence fondamentale, de préférence par un doublement de la fréquence, au-dessus de certaines valeurs limites de la densité de puissance, qui correspond à la valeur moyenne d'une impulsion, et il est prévu, dans le cas de la disposition du cristal à l'extérieur du résonateur, des moyens (23), par lesquels les pics d'intensité de fréquence multipliée sont éloignés du chemin du rayon de la longueur d'onde de base à introduire dans le guide d'ondes lumineuses.

2. Dispositif suivant la revendication 1, caractérisé en ce que le cristal (4) disposé dans le chemin du rayon d'entrée du laser solide est disposé à l'extérieur du résonateur (1,5) laser et est disposé par rapport à un guide d'ondes lumineuses (17), de telle sorte que les pics d'intensité d'une impulsion laser soient doublés en fréquence et soient éloignés du trajet du rayon par l'intermédiaire d'un séparateur (23) de rayon dichroïque, de sorte que seule la longueur d'onde fondamentale, qui ne dépasse plus à aucun instant certaines densités de puissance critiques, soit couplée dans le guide d'ondes lumineuses (17).

3. Dispositif suivant la revendication 1, caractérisé en ce qu'il est utilisé comme laser un grenat d'yttrium-scandium-gadolinium dopé au Cr-Er fonctionnant à une longueur d'onde de 2,78 µm.

4. Dispositif suivant la revendication 1, caractérisé en ce qu'il est utilisé comme laser un laser Er:YAG fonctionnant à 2,94 µm.

5. Dispositif suivant la revendication 2, caractérisé en ce qu'il est utilisé comme cristal (4) de l'iodate de lithium ou du sulfure d'argent et de gallium.

6. Dispositif suivant la revendication 1, caractérisé en ce que le rayonnement actif impulsionnel est transmis par un guide optique d'ondes lumineuses (17) sous forme d'une fibre ou d'un dispositif multi-fibres en fluorure de zirconium.

7. Dispositif suivant la revendication 6, caractérisé en ce que le guide d'ondes lumineuses (17) est entouré par une matière plastique (18) viscoélastique et est enrobé dans une gaine (19) résistant à la torsion.

8. Dispositif suivant la revendication 6, caractérisé en ce que le guide d'ondes lumineuses (17) est muni du côté du laser et du côté applicateur d'un accouplement (8, 10) rapide en rotation, qui empêche l'application de forces de torsion sur la partie flexible du guide d'ondes lumineuses.

9. Dispositif suivant la revendication 6, dans lequel le guide d'ondes lumineuses (17) peut être relié à une pièce tenant dans la main, qui comporte du côté de sortie de la lumière une pièce (14) d'extrémité d'applicateur, caractérisé en ce qu'il est prévu dans le trajet du faisceau de la lumière laser des moyens (25, 29, 30), qui empêchent que les produits d'ablation produits par le rayonnement ne puissent pas détruire les surfaces optiques de la pièce (14) d'extrémité d'applicateur.

10. Dispositif suivant la revendication 9, caractérisé en ce que la pièce (14) d'extrémité d'applicateur est sous forme du guide optique creux, qui est relié à un réservoir (25) de gaz communiquant avec une conduite (15) d'amenée de gaz.

11. Dispositif suivant la revendication 10, caractérisé en ce que le réservoir (25) de gaz est fermé, au moins du côté du laser, par une fenêtre optique (29), de préférence en mica.

12. Dispositif suivant la revendication 11, caractérisé en ce que la pièce (14) d'extrémité d'applicateur est une pointe (30) de saphir de préférence de forme conique.

13. Dispositif suivant la revendication 1, caractérisé en ce qu'il est prévu des moyens (15, 16, 38, 39), à l'aide desquels des gouttelettes d'aérosol peuvent être insufflées dans la zone active du rayonnement laser et il est prévu d'autres moyens (34 à 40), à l'aide desquels la relation air-fluide et la quantité de fluide en fonction de l'énergie des impulsions laser et du taux de répétition des impulsions peuvent être commandées.
